# EUROPEAN PATENT SPECIFICATION

(11) **EP 3 595 655 B1**
(45) Date of publication and mention of the grant of the patent: **07.02.2024**
(21) Application number: 18712537.2
(22) Date of filing: 14.03.2018
(51) Int. Cl.: A61K 31/4045, A61K 9/14, A61K 47/02, A61K 47/40, A61P 43/00

(54) **THERAPEUTIC DOSAGE AND REGIMEN FOR MELATONIN**
THERAPEUTISCHE DOSIERUNG UND REGIMEN FÜR MELATONIN
DOSAGE ET RÉGIME THÉRAPEUTIQUE DE MÉLATONINE

(30) Priority: 17.03.2017 EP 17161472
(43) Date of publication of application: 22.01.2020
(73) Proprietor: Chiesi Farmaceutici S.p.A., 43122 Parma (IT)
(72) Inventor: FACCHINETTI, Fabrizio, 43122 Parma (IT); AQUINO, Giancarlo, 43122 Parma (IT); ROBERTSON, Nicola Jayne, 43122 Parma (IT)
(74) Representative: Bianchetti & Minoja with Trevisan & Cuonzo IPS SRL
(86) International application number: PCT/EP2018/056423
(87) International publication number: WO 2018/167162

(56) References cited:
- WO-A1-2016/139635
- WO-A2-2013/068565
- US-A1- 2015 258 064
- ROBERTSON N ET AL.: "Melatonin augments hypothermic neuroprotection in a perinatal asphyxia model", BRAIN, vol. 136, no. 1, 2013, pages 90-105, XP002773457, cited in the application
- K JANE HASSELL ET AL: "New horizons for newborn brain protection: enhancing endogenous neuroprotection", ARCHIVES OF DISEASE IN CHILDHOOD. FETAL AND NEONATAL EDITION, vol. 100, no. 6, 10 June 2015 (2015-06-10) , pages F541-F552, XP055403764, GB ISSN: 1359-2998, DOI: 10.1136/archdischild-2014-306284
- MOHAMED HENDAUS ET AL: "Melatonin in the management of perinatal hypoxic-ischemic encephalopathy: light at the end of the tunnel?", NEUROPSYCHIATRIC DISEASE AND TREATMENT, vol. Volume 12, 1 September 2016 (2016-09-01), pages 2473-2479, XP055403754, DOI: 10.2147/NDT.S115533
- Jeffrey Roy Johns ET AL: "An intravenous injection of melatonin: formulation, stability, pharmacokinetics and pharmacodynamics", , 1 January 2012 (2012-01-01), XP055404227, Retrieved from the Internet: URL:http://www.aaspjournal.org/pdf_fullpap er/vol01no01_40-51.pdf

## Description

### TECHNICAL FIELD

The present invention relates to a therapeutic dosage and regimen for the parenteral administration of melatonin-based formulations to neonates affected by a brain injury disease due to birth asphyxia.

### BACKGROUND OF THE INVENTION

Neonates, especially if born prematurely, are very susceptible to free radical oxidative damage. In fact infants at birth are: a) naturally exposed to hyperoxic challenge due to the transition from the hypoxic intrauterine environment to extrauterine life, and this gap is even more significant for neonates that require supplemental oxygen during resuscitation in the delivery room; b) they are more susceptible to infection, especially if born prematurely; c) they have reduced antioxidant defences; d) they possess high levels of free iron that enhances the Fenton reaction causing the production of highly toxic radicals. Oxidative stress likely contributes also to the severity of several neonates diseases as it may affect a variety of organs, often simultaneously, giving rise to different signs according to the organ most damaged. Said diseases include bronchopulmonary dysplasia/chronic lung disease (BDP/CLD), retinopathy of prematurity (ROP), and necrotizing enterocolitis (NEC). Subsequently, it became clear that free radicals are involved in perinatal brain injury as well as in influencing the ductus arteriosus and pulmonary circulation.

In order to counteract free radicals damage many strategies to increase the antioxidant capabilities in term and preterm infants have been proposed and several medications have been experimented with contrasting results.

N-[2-(5-Methoxy-1H-indol-3-yl)ethyl] acetamide, known as melatonin, is an endogenous substance mainly synthesized in the pineal gland from the neurotransmitter serotonin. Melatonin plays a key role in a variety of important physiological functions, including regulation of circadian rhythms, as well as visual, reproductive, cerebrovascular, neuroendocrine, and neuro-immunological actions. Melatonin is a highly effective free-radical scavenger which also enhances the antioxidant potential of the cell by stimulating the synthesis of antioxidant enzymes and by augmenting glutathione levels. Melatonin is also known to counteract cellular energy depletion by preserving mitochondrial homeostasis and protects mitochondrial ATP synthesis by stimulating Complexes I and IV activities. Moreover, melatonin has been shown to attenuate microglial activation and neuroinflammatory responses which are typically associated with hypoxic-ischemic insults. Beside its well documented neuroprotective efficacy (see for example Balduini W et al J Matern Fetal Neonatal Med. 2012, 25(S1), 119-124), melatonin is an interesting drug, because of its safety profile and its ability to cross all physiological barriers and to reach subcellular compartments.

In light of these properties, during the last decade, melatonin has started to be considered an attractive neuroprotective agent in perinatal asphyxia.

On the other hand, the oral bioavailability of melatonin is low and very variable. Furthermore, melatonin is poorly soluble in water and degrades quickly. In the prior art, evidences were indeed reported indicating that melatonin in aqueous solution gradually loses potency at all pH values and is not stable when exposed to light or oxygen. In this respect, it is also well known that some stabilizers and/or preservatives may have the potential to cause toxicological problems, especially in the infant population.

Furthermore, the pharmacokinetic profile of melatonin in infants differs from that of adults; therefore dosage of melatonin for term or preterm infants cannot be extrapolated from adult studies.

WO 2012/156565, WO 2013/068565 and WO 2015/135997 discloses different injectable formulations comprising melatonin for use for the treatment of neonatal brain injury. However, as for as suitable therapeutic dosage and regimen are concerned, only generic indications are provided. WO2015/13997 discloses doses of 1-40 mg/Kg of melatonin, preferably administered during a six hour window.

WO 2016/139635 discloses a pharmaceutical suitable for parenteral administration wherein melatonin have a concentration of 0.2-0.4 mg/ml, but the dosage to be used for the treatment of neonatal brain injury is not reported.

Robertson N et al (Brain 136(1), 2013, 90-105) have shown that melatonin administered intravenously to newborn piglets increases hypothermic neuroprotection at a daily dose of 5 mg/kg/h over six hours (30 mg/kg). Nevertheless, the formulation utilized in this study is not suitable for administration in human neonates.

Furthermore, said formulation was administered at 10 minutes after resuscitation, a time for intervention that has no concrete application in the clinical practice given the required time for handling the neonates in the delivery rooms and/or in the Neonatal Intensive Care Units (NICUs).

Further investigator initiated trials are on-going. For example, Jerez-Calero A et al in J Ped Neonatal Individ Med 6(1), 2017 disclosed to administer melatonin intravenously at a dose of 5 mg/kg every 24 hours (3 doses) before the first 6 hours of life, but it is silent about the criticality of the rate of delivery.

On the other hand, the applicant found that the formulation should be administered not only not later than six hours from the resuscitation, but that it is also very important the rate of delivery.

In view of these drawbacks of the prior art, it would be highly advantageous to provide an efficacious therapeutic regimen for the parenteral administration of melatonin to neonates affected by a brain injury disorder.

The issue of a safe and effective parenteral delivery of therapeutic doses of melatonin to neonates is solved by the present invention.

### SUMMARY OF THE INVENTION

In a first aspect, the invention refers to a safe melatonin pharmaceutical formulation for use in the treatment of brain injuries caused by birth asphyxia in a neonate, wherein said formulation is administered by parenteral infusion at a daily dose of 10 to 20 mg/kg, characterized in that the administration is started not earlier than 45 minutes but not later than 4 hours, more preferably between 1 hour and 2 hours, from resuscitation of said neonate and the time period for the infusion is comprised between 30 minutes and 4 hours.

In a preferred embodiment, the safe pharmaceutical formulation is a ready-to-use formulation comprising melatonin dissolved in a suitable aqueous vehicle or is a melatonin powder to be reconstituted with the same or a different suitable aqueous vehicle.

Also disclosed is a package comprising a safe melatonin pharmaceutical formulation in form of either ready-to-use aqueous solution or powder to be reconstituted in a suitable aqueous vehicle, in combination with instructions to parenterally administer by infusion said pharmaceutical formulation to a neonate affected by a brain injury disorder due to birth asphyxia at a daily dose of 10 to 20 mg/kg, characterized in that the administration is started not earlier than 30 minutes but not later than 6 hours, preferably comprised between 45 minutes and 4 hours, more preferably between 1 hour and 2 two hours, from resuscitation of said neonate and the time period for the infusion is no longer than 6 hours.

Also disclosed is a safe melatonin pharmaceutical formulation for use in the treatment of brain injuries caused by birth asphyxia in a neonate, wherein said formulation is administered by parenteral infusion at a daily dose of 10 to 20 mg/kg, for 1 to 30 days characterized in that the first administration is started not earlier than 30 minutes but not later than 6 hours from resuscitation of said neonate and the time period for the infusion of each administration is no longer than 6 hours.

### DEFINITIONS

With reference to melatonin, the terms "drug", "active ingredient" and "active substance" are used interchangeably.

The terms "neonates", "newborns" and "infants" are used interchangeably.

With the term "neonatal resuscitation" it is meant the process performed in Neonatal Intensive Care Units (NICUs) or in the delivery room to counteract the signs of birth asphyxia.

The term "safe" means a pharmaceutical formulation suitable for injection able of satisfying the injectability criteria for medicinal products, well tolerated by neonates, and devoid of excipients that could be harmful, antigenic or toxic for these patient population.

For a ready-to-use formulation, the expression "physically stable" refers to a formulation that, under accelerated conditions (40°C ± 2°C, 75% ± 5% relative humidity), exhibits substantially no growth in particle size during storage for at least three months, preferably six months, is readily redispersible, and upon redispersion, neither agglomerates are nor quick separation from the aqueous vehicle are observed so as to prevent reproducing dosing of the active ingredient.

The expression "chemically stable" refers to a formulation that, upon storage, meets the requirements of the EMEA Guideline CPMP/QWP/122/02 referring to "Stability Testing of Existing Active Substances and Related Finished Products".

The term "therapeutically effective amount" means the amount of the active ingredient, that, when delivered to neonates, provides the desired biological effect.

The term "prophylaxis" refers to the therapeutic use for reducing the occurrence of a disease.

The term "treatment" refers to the therapeutic use for palliative, curing, symptom-allievating, symptom-reducing, disease regression-inducing therapy.

### FIGURES

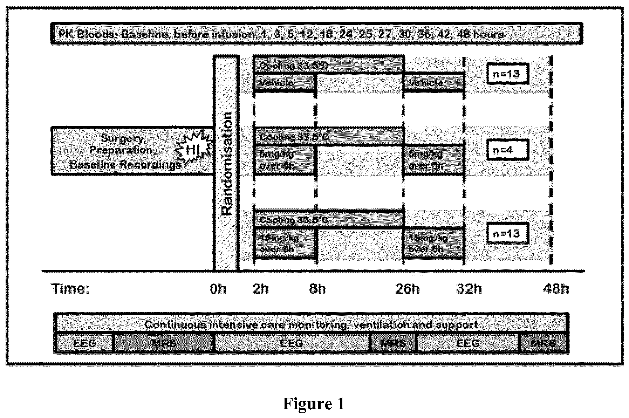

**Figure 1** - Study time line. Following baseline data acquisition, piglets underwent cerebral hypoxia-ischemia. At the end of hypoxia-ischemia piglets were randomised to: (i) Vehicle (at 2h and infused over 6h) plus cooling to 33.5°C for 24h starting at 2h; (ii) Melatonin 5 mg/kg at 2h and 26h and infused over 6h and cooling; (iii) Melatonin 15 mg/kg at 2h and 26h and infused over 6h and cooling. Piglets were maintained under intensive care for 48h following hypoxia-ischemia prior to euthanasia. MRS was acquired at baseline, during hypoxia-ischemia, for the first 60 minutes after hypoxia-ischemia at 24 and 48h. EEG was acquired at baseline and in between the MRS acquisitions.

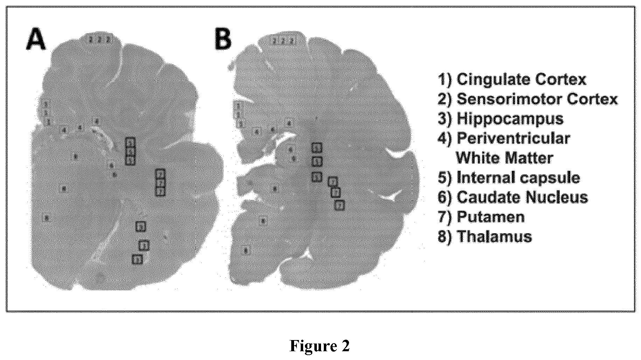

**Figure 2** - The two brain levels A and B and 3 fields per region for TUNEL quantification. The thin-line squares are those regions sampled with MRS voxels while the bold-line squares are brain region not sampled within MRS voxels.
**Figures 3**

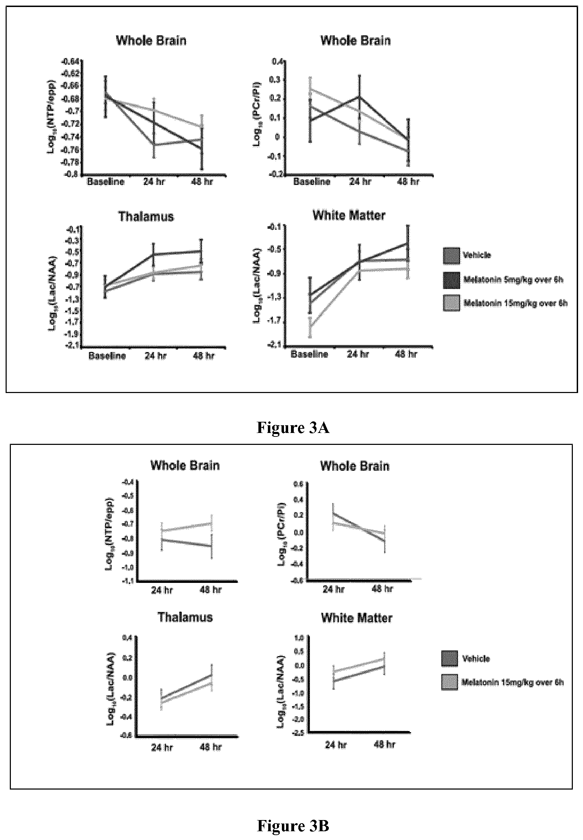

   Figure 3A shows 9.4T magnetic resonance spectroscopy of the brain at baseline, 24 and 48h after hypoxia-ischaemia. Least squares means plots with SEM error bars for the NTP/epp and PCr/Pi in whole forebrain and Lac/NAA in the thalamus and white matter. There was no difference apart from 24h NTP/epp higher in the 15 mg/kg Melatonin group (p=0.038).
   Figure 3B shows 3T magnetic resonance spectroscopy of the brain at 24 and 48h after hypoxia-ischaemia. SEM error bars for the NTP/epp and PCr/Pi in whole forebrain and Lac/NAA in the thalamus and white matter; non-overlapping bars show evidence of a significant difference. There was no difference in any ratio at 24 and 48h.

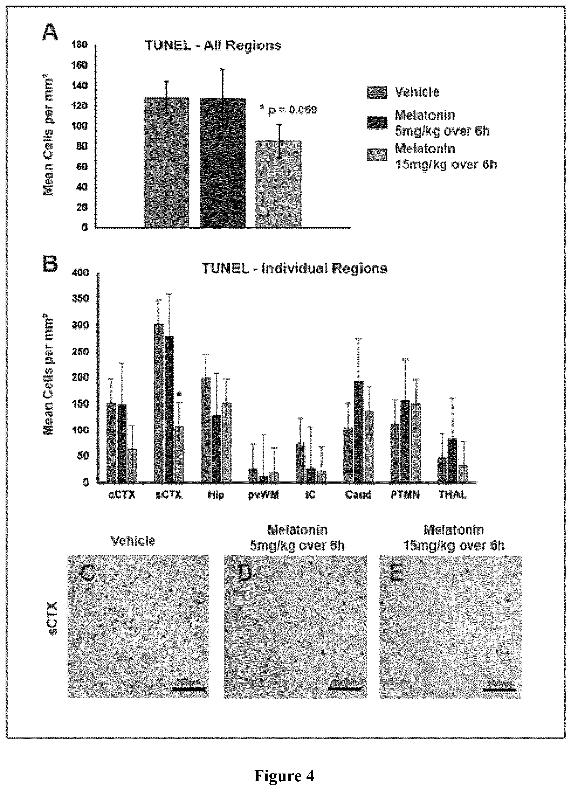

**Figure 4** - Mean TUNEL counts for vehicle, 5 mg/kg over 6 h Melatonin & 15 mg/kg over 6h. (A) Across all brain regions. There is a trend towards reduced TUNEL positive cells in the high dose melatonin group (p=0.069). (B) Across individual brain regions. There was a significant reduction in TUNEL positive cells in the high dose melatonin group compared to vehicle in the sensorimotor cortex (p=0.003). (C-E) Representative TUNEL sections of the sensorimotor cortex taken at x20 magnification.

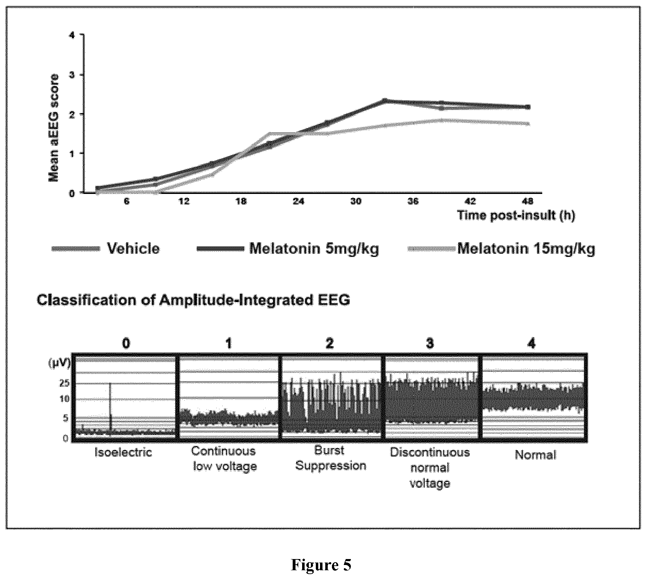

**Figure 5** - Amplitude-integrated EEG (aEEG). There was no difference in the mean hourly aEEG scores in any group at any timepoint.

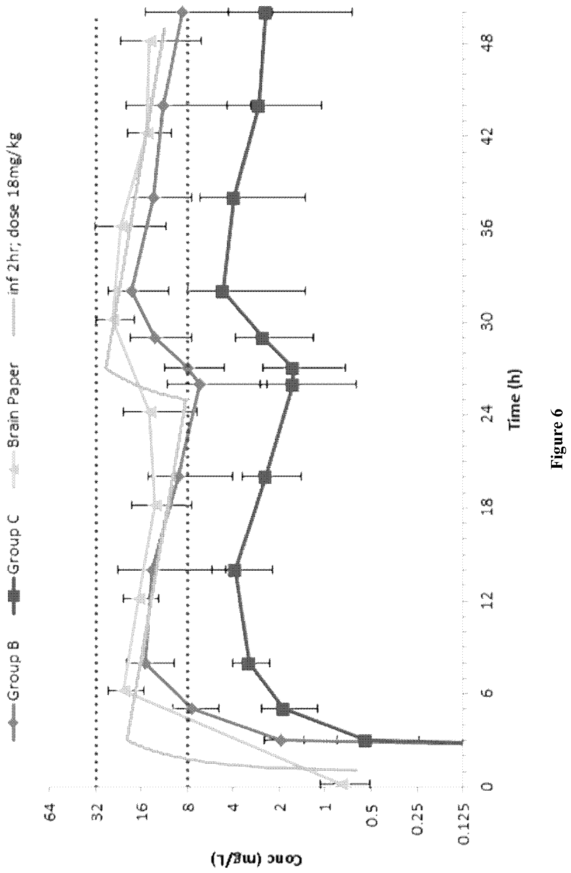

**Figure 6** - Mean plasma profiles of the historical Brain study (dotted lines and triangles-line), low dose Melatonin (squares-line) and high dose Melatonin (diamonds-line). The modelled PK for 18mg/kg over 2h is shown by the light grey line.

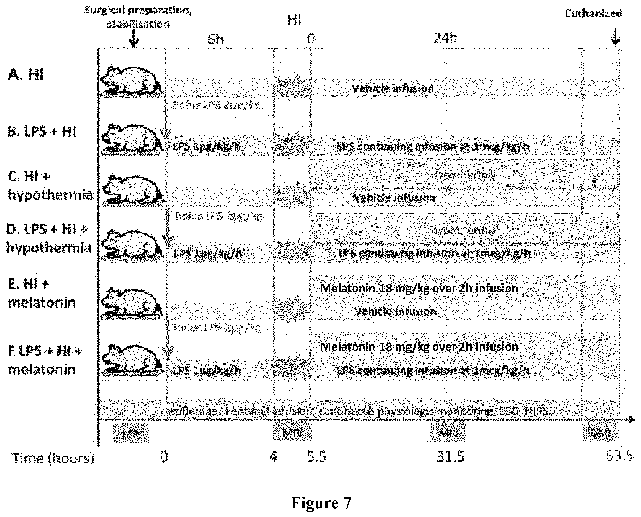

**Figure 7** - Neuroprotection efficacy studies comparing hypothermic versus Melatonin neuroprotection in pure HI and inflammation sensitised HI. Primary endpoints for the efficacy studies are (i) Cerebral Lac/NAA rise over the 48h after HI (ii) TUNEL positive cells in 11 brain regions. Melatonin dose is comprised between 15 and 18 mg/kg and time of infusion between 2 and 3 hours.

### DETAILED DISCLOSURE OF THE INVENTION

Thanks to its antioxidant activity and to its other pharmacological properties, melatonin could be successfully used in the prophylaxis and/or treatment of certain neonatal diseases.

However, the formulation shall be administered within a suitable time from the resuscitation, otherwise the efficacy of melatonin would decrease.

Furthermore, it shall be administered at a suitable delivery rate in such a way that the time period of the infusion is no longer than 4 hours.

It has indeed been found that, in order to achieve as faster as possible the effective plasmatic concentration of the active ingredient, that the applicant has determined to be comprised between 10 and 100 mM, the formulation shall be administered at a delivery rate in such a way as that the period of infusion is comprised between 30 minutes and 4 hours, preferably between 1 hour and 2 hours.

Therefore, the aim of the present invention is to provide an efficacious therapeutic regimen for the parenteral administration by infusion of a melatonin-based pharmaceutical formulation to neonates affected by a brain injury disorder.

In a preferred embodiment of the invention, the safe pharmaceutical formulation is delivered by a proper infusion pump.

Advantageously, said safe pharmaceutical formulation of melatonin shall be administered at a daily dose of 10 to 20 mg/kg, more advantageously between 12 and 18 mg/kg, preferably of 15 mg/kg, starting not earlier than 30 minutes but not later than 4 hours from resuscitation of the neonate.

The safe formulation comprised melatonin dissolved in an aqueous vehicle.

It could be in form of a dry powder to be dissolved extemporaneously before use or in form of ready-to-use formulation.

In case it is dispensed as dry powder to be redissolved, it may be prepared according to methods known to the skilled person in the art, such as freeze-drying, and it may be as a kit comprising a) the freeze-dried pharmaceutical formulation; b) a pharmaceutically acceptable aqueous vehicle; c) container means for containing the pharmaceutical formulation, and the aqueous vehicle.

As freeze-dried product, the formulation may contain cryo-protectant agents such as mannitol.

Melatonin could be used as a free base or in form of any pharmaceutically acceptable salt and/or solvate thereof.

Advantageously, the concentration of melatonin shall be comprised between 1 and 10 mg/ml, more advantageously between 2 and 8 mg/ml, preferably between 2.5 mg/ml and 5 mg/ml.

In a particular embodiment of the invention, the concentration of melatonin shall be of 5 mg/ml, while in another particular embodiment shall be of 2.5 mg/ml.

Said formulations to be safe should contain pharmaceutically acceptable excipients and/or solubilizing agents suitable for parenteral use and selected from those well tolerated by neonates.

For example, the formulation may contain propylene glycol or polyethylene glycol (PEG) as a co-solvent as reported in WO 2012/156565.

Alternatively, as solubilizing agent, the formulation may contain sulfobutylether-β-cyclodextrin currently sold under the trademark of Captisol^{™} (Ligand Pharmaceuticals Inc, San Diego, California) or hydroxypropyl-β-cyclodextrin sold under the trademark of Trappsol^{®} Cyclo^{™} (CTD Holdings Inc, Alachua, Florida), preferably sulfobutylether-β-cyclodextrin.

In some embodiments of the invention, the formulation may contain as solubilizing agent, sulfobutylether-β-cyclodextrin in admixture with PEG 300 or PEG 400 in ratios ranging from 85:15 to 95:5 w/w, preferably 90:10 w/w.

Any pharmaceutically acceptable aqueous vehicle suitable for parenteral administration to neonates could be used, for example water for injection (WFI). Otherwise, a saline aqueous solution (0.9% w/v sodium chloride) or a glucose solution could be utilised at a proper concentration that shall be adjusted by the skilled person in the art. In some embodiments, a half-saline aqueous solution (0.45% w/v sodium chloride) could be preferable.

In other embodiments, a glucose aqueous solution at a concentration of 5% or 10% w/v could advantageously be used.

The safe pharmaceutical formulation may comprise other excipients, for instance tonicity agents and pH buffers such as acetate, phosphate or citrate buffers, tris(hydroxymethyl)aminomethane-HCl (Tris-HCl), preferably phosphate or Tris-HCl, more preferably phosphate buffer.

Advantageously the pH of the pharmaceutical formulation may be comprised between 6.5 and 7.5, preferably 7.0 ± 0.1.

Since the safe pharmaceutical formulation should be suitable for parenteral administration, its osmoticity is of particular importance. Accordingly, the formulation of the invention shall have an osmolality of less than 600 mOsm/kg advantageously from 150 mOsm/kg to 500 mOsm/kg, more advantageously from 200 to 400 mOsm/kg, preferably from 250 to 350 mOsm/kg.

The person skilled in the art could easily convert osmolality values in osmolarity values by determining the density of the formulation.

Optionally, the above formulation is sterile.

Sterilization can be achieved according to known methods. For example, the powder may be sterilized by gamma-irradiation, while the pharmaceutical formulation ready-to-use may be sterilized by filtration or by autoclaving treatments.

In a preferred embodiment of the invention, the formulation comprises melatonin at a concentration of 2.5 or 5.0 mg/ml.

In a particular embodiment of the invention, the safe formulation has the following composition:
5.0 mg/ml melatonin, 50 mg/ml Captisol^{™}, 0.2 mg/ml phosphate buffer (10 mM), optionally 4.5 mg/ml sodium chloride, water for injection.

In a further particular embodiment, the safe formulation has the following composition:
2.5 mg/ml melatonin, 25 mg/ml Captisol^{™}, 0.2 mg/ml phosphate buffer (10 mM), optionally 4.5 mg/ml sodium chloride, water for injection.

The above formulations could be filled in suitable containers, for example vials made of glass of amber colour, and are particular suitable for the administration of melatonin according to the therapeutic regimen of the invention.

The capacity of said vials may vary from 2 to 10 ml, preferably not higher than 6 ml.

However, the skilled person in the art, depending on the composition of the safe formulation shall choose the concentration and the capacity in order to delivery it in a period comprised between 30 minutes and four hours, more preferably between 1 hour and two hours.

For example, if the daily dose to be administered is 15 mg/kg, a formulation in which the concentration of melatonin is 2.5 mg/ml will be filled in a vial of 6 ml to be delivered in a time of 3 hours at a delivery rate of 5 mg/kg/hr.

As a further Example, if the daily dose to be administered is 20 mg/kg, a formulation in which the concentration of melatonin is 5 mg/ml will be filled in a vial of 4 ml to be delivered in a time of 2 hours at a delivery rate of 10 mg/kg/hr.

The pharmaceutical formulations of the invention are for use in the prophylaxis and/or treatment of any neonatal brain disorder disease due to birth (perinatal) asphyxia.

These diseases includes and Perinatal Arterial Stroke (PAS), and Periventricular Leucomalakia (PVL), and hypoxic - ischemic encephalopathy (HIE), preferably hypoxic - ischemic encephalopathy (HIE).

The hypoxic- ischemic encephalopathy is indeed one of the major reasons of neonatal death and neurological disability in children. The estimated incidence of this disease is of about 1- 2/1000 infants born at term and increases up to 60% among premature infants weighing less than 1500 grams. A percentage comprised between 20 and 50% of asphyxiated infants who develop a hypoxic-ischemic encephalopathy unfortunately are destined to die in the neonatal period, while about 25% of the survivors have severe neurological disabilities such as cerebral palsy, mental retardation, epilepsy, and learning disorders.

The therapeutic regimen of the invention could be applied to both term and pre-term neonates having a gestational age equal to or lower than 37 weeks.

When the therapeutic regimen of the invention is directed to protect the neonates from neurological damages, the safe formulation may be administered for only 1 day.

In another embodiment, when it is directed to restore a neurological function, the administration of the formulation may be administered repeatedly, for example from 1 to up 30 days. In some embodiments, the formulation could be administered from 1 to 7 days, while in another embodiment, from 1 to 14 days.

Within the limit of the claimed therapeutic regimen, the physician shall adjust the dosage and/or the duration of the treatment depending of the gestational age and weight of the newborn as well as of the severity of the disease as long as the first administration is started not earlier than 45 minutes but not later than 4 hours from resuscitation of said neonate and the time period for the infusion of each administration is no longer than 4 hours.

Nowadays, hypothermia is recognized as an efficacious treatment modality for perinatal asphyxia and HIE. Accordingly, the safe formulation according the therapeutic regimen of the invention could be applied in combination with hypothermia, leading to a greater cerebral neuroprotective effect than hypothermia alone, thus improving the immediate and long term clinical outcome.

Within the limit of the claimed dosage interval, the physician shall adjust the dosage and the frequency of administration depending of the gestational age and weight of the newborn as well as of the severity of the disease.

The invention is illustrated with reference to the following examples.

### EXAMPLE 1 - Determination of the formulations

The first set of experiments was aimed to assess the possibility to solubilise melatonin with different quantity of sulfobutylether-β-cyclodextrin (Captisol^{™}).

The test was performed by the addition of melatonin. In the solution containing the Captisol at different concentrations up to presence of undissolved material on the bottom; the solution was filtered and analysed.

These tests were performed with three different vehicles: water for injection (WFI), glucose 10% solution and phosphate buffer.

In a second set of experiments, PEG with different molecular weights (PEG 300 and PEG 400) was added to verify the possibility to reduce the amount of Captisol used for the dissolution.

The results of the first set of experiments, were obtained measuring the assay in formulation at the higher solubility limits having on the bottom some undissolved particles. These data were used to understand the amount of Captisol necessary to reach 5 mg/ml concentration and avoiding the edge effect. Captisol concentration of 5% was identified as a suitable one, and confirmed by the other results.

From this preliminary test, it was evident that the presence of glucose or buffer as vehicle does not influence the solubility of the melatonin; on the contrary, the presence of glucose or buffer contributes to produce osmolality and this should be taken in consideration in order define a formulation with a suitable osmolality value of the solution.

With regards to the addition of other excipients (second set of experiments), the results demonstrate that is possible to reduce the quantity of Captisol, but 10% of PEG is required.

Considering the paediatric use of these formulations, it was decided to formulate the product with as less as possible excipients; consequently the PEG was skipped and the formulation with Captisol alone was selected.

Some trials were also performed in order to evaluate the possibility and opportunity to add a buffer to the formulation.

In particular, it was consider important to understand how maintain the pH stable in the target range.

Samples were prepared with 10 mM phosphate buffer and different Captisol quantity, as reported in Table 1.

**Table 1 - Comparison of the results obtained during the preliminary solubility trial among solution with and without buffer.**

| Vehicle | Captisol | pH | Osmolality | Density |
|---|---|---|---|---|
| 10 mM buffer phosphate pH7 | 5% | 6.79 | 151 mOsmol/Kg | 1.022 g/ml |
| 10 mM buffer phosphate pH7 | 7.5% | 6.76 | 223 mOsmol/Kg | 1.033 g/ml |
| 10 mM buffer phosphate pH7 | 10% | 6.75 | 302 mOsmol/Kg | 1.043 g/ml |
| 10 mM buffer phosphate pH7 | 12.5% | 6.65 | 361 mOsmol/Kg | 1.053 g/ml |
| Water for injection | 5% | 6.67 | 129 mOsmol/Kg | 1.021 g/ml |
| Water for injection | 10% | 5.50 | 275 mOsmol/Kg | 1.042 g/ml |

The results in Table 4 indicate that a 10 mM buffer is suitable to maintain pH in the desired range.

In addition, the recorded osmolality values recorder indicate the contribution to formulation osmolality value by phosphate buffer (about 20-30 mOsmol/Kg) and by the different Captisol quantity. In the light of these results, it was decided to use the 10 mM phosphate buffer solution as vehicle.

### EXAMPLE 2 - Preparation of a safe formulation according to the invention

The unitary composition of formulation 1 is reported in Table 2.

**Table 2 - Safe formulation suitable for clinical use**

| Ingredient | % | Conc |
|---|---|---|
| Melatonin | 0.5 | 5.0 mg/ml |
| Captisol | 5.0 | 50 mg/ml |
| Na₂HPO₄ heptahydrate | 0.058 | 0.58 mg/ml |
| NaH₂PO₄ monohydrate | 0.155 | 1.55 mg/ml |
| Water for injection q.s. to 100 ml | | |

The pH turned out to be 7.0 ± 0.3.

The osmolality turned out to be of about 150 mOsm/Kg.

### EXAMPLE 3 - Stability of the formulation of Example 1

A formulation as prepared in example 1 was stored for six months under accelerated conditions, i.e. 40° ± 2 C and 75 ± 2% relative humidity, in order to evaluate its physical and chemical stability.

The assay of melatonin and its related substances and impurities were performed by HPLC. The following parameters were also tested: pH, and appearance.

The results are shown in Table 3.

**Table 3 - Stability of formulation according to Example 2**

| Formulation of Example 2 | pH | Assay (mg/ml) | Related substances (%) | Total impurities (%) | % vs INITIAL | Appearance |
|---|---|---|---|---|---|---|
| Initial (t = 0) | 6.75 | 5.41 | 0.02 | 0.07 | 100 | Clear and colourless |
| t = 1 months | 6.76 | 5.22 | 0.03 | 0.07 | 96 | Clear and colourless |
| t = 2 months | 6.9 | 5.14 | 0.02 | 0.07 | 95 | Clear and colourless |
| t = 3 months | 6.9 | 5.30 | 0.02 | 0.06 | 98 | Clear and colourless |
| t = 6 months | 6.8 | 5.31 | 0.02 | 0.08 | 98 | Clear and colourless |

The results reported in Table 3 show that no appreciated degradation can be observed after at least 6 months when the formulation was stored under accelerated conditions. The assay variation was within ± 5%.

The formulation also turned out to be physically stable.

### EXAMPLE 4 - Intravenous administration of the melatonin formulation of Example 2 at a dose of 15 mg/kg after 2 hours from transient hypoxia-ischaemia

Objective: To assess neuroprotection of intravenous melatonin (15 mg/kg) infused slowly over 6h (2.5 mg/kg/h) initiated 2 h after hypoxia-ischaemia in a piglet model of neonatal asphyxia (Fig. 1). Primary outcome measures were TUNEL immuno-labelling of cell death over 9 brain regions (Fig. 2). Secondary outcomes were amplitude-integrated electroencephalography(aEEG) recovery, and magnetic resonance spectroscopy (MRS) biomarkers (NTP/epp and lactate/N acetyl aspartate).

Design/Methods: as depicted in Fig. 1, 26 piglets were randomized after hypoxia-ischemia to vehicle (n=13) and melatonin (6h infusion at 2h and 26h after insult, n=13). Both groups were cooled (33.5°C 2-26h). Hypoxia-ischemia was induced inside the MR scanner by remotely inflating the vascular occluders around both common-carotid arteries, and simultaneously reducing FiO2 to 6% (vol/vol) (Robertson et al., Brain 2103). During hypoxia-ischemia the β-NTP peak height was continuously monitored using in-house Matlab (Mathworks) software. At the point at which β-NTP had fallen to 50% of its baseline value, FiO2 was increased to 9%. When β-NTP fell to 40% baseline height the inspired oxygen fraction was titrated to keep the β-NTP peak height between 30% and 40% of its original height for a period of 12.5 minutes. At the end of hypoxia-ischemia the carotid arteries were de-occluded and the FiO2 returned to 21%. Insult severity was calculated Anesthesia, intensive care support, aEEG and physiological monitoring were maintained for 48h when cell death (TUNEL staining for apoptotic cells) was scored over 9 brain regions. Magnetic resonance: ¹H MRS and ³¹P MRS were acquired at 24 and 48h. The head was immobilized in a stereotactic frame for MRS acquisition. Piglets were positioned within the bore of 9.4 Tesla Agilent MR scanner. ¹H and ³¹P MRS data were acquired at baseline (for all piglets n=30) and at 24h (vehicle n=11, 5 mg/kg Mel-N n=4, 15 mg/kg Mel-N n=13) and 48 h (vehicle n=8, 5 mg/kg Mel-N n=4, 15 mg/kg Mel-N n=12) after cerebral hypoxic-ischemia. After surgical preparation, multichannel six-lead EEG monitoring (Nicolet, Care Fusion, Wisconsin, USA) was acquired at baseline and throughout the periods between MRS data acquisitions. Filtered amplitude-integrated EEG recordings were classified according to the pattern classification. A score of 0 was flat trace; 1, continuous low voltage; 2, burst suppression; 3, discontinuous normal voltage; and 4, continuous normal voltage, at baseline and then every hour after hypoxia-ischemia. Blood was sampled at baseline, before infusion, 1, 3, 6, 12, 18, 24, 25, 27, 30, 36, 42, 48h after hypoxia-ischaemia. CSF was sampled at 48h. Blood samples were collected in lithium/heparin tubes and centrifuged immediately after collection. Plasma was separated and then stored at - 20°C before analysis. Melatonin was routinely measured by direct radioimmunoassay. Piglets were euthanized at 48 h.

Results: At 24h post insult, melatonin ameliorated the NTP/ePP decline induced by hypoxic-ischemic insult in comparison to vehicle (p=0.038). aEEG recovery rate was similar between groups (Fig. 3).

Melatonin treatment started 2h after hypoxia-ischaemia, reduced cell death in the sensorimotor cortex (p<0.003) compared to vehicle (Fig. 4). There was a trend of reduced cell death across the whole brain (p=0.068) in the melatonin-treated group (Fig. 4). There was no difference in the group mean hourly aEEG scores between groups at any time point (Fig. 5). Melatonin-treated piglets showed plasma levels within the expected therapeutic range (Robertson N et al, Brain 136(1), 2013, 90-105). The dose of melatonin and the type of formulation utilized in this study did not affect the physiological parameters monitored, including mean arterial pressure and inotrope use.

Conclusion(s): Melatonin brain protection following hypoxia-ischaemia in the newborn piglet is dependent on dose and timing of administration; no protection was seen with 5 mg/kg/24h melatonin, a trend to overall reduced TUNEL positive cells was seen with 15 mg/kg/24h and significant protection was seen in the sensorimotor cortex. In newborn piglets subjected to hypoxia-ischaemia, intravenous infusion of a high dose (15 mg/kg) of melatonin formulation according to Example 1 is well tolerated and augments hypothermic neuroprotection in sensorimotor cortex when initiated 2h after the insult.

Discussion: Melatonin brain protection following hypoxia-ischaemia in the newborn piglet was dependent on dose and timing of administration; with melatonin administered at 2h after hypoxia-ischaemia over a period of 6h, no protection was seen with 5 mg/kg/24h melatonin and a trend to overall brain reduced TUNEL positive cells was seen with 15 mg/kg/24h. Significant brain protection was seen only in the sensorimotor cortex with 15 mg/kg/24h Mel-N given at 2h. Importantly, Cmax with the 15 mg/kg Mel-N started at 2h was reached at 8h after hypoxia-ischaemia. It is likely that earlier and faster administration of melatonin is needed to more rapidly reach the putative neuroprotective concentration of melatonin in plasma and achieve better brain protection after hypoxia-ischaemia. As an example in Figure 6 (orange line), pharmacokinetic modelling has shown that a dose of 18 mg/kg over 2h started at 1h, repeated every 24h would lead to a Cmax at 3h, i. e. 5h earlier than what observed in the study described in this Example.

### EXAMPLE 5 - A neuroprotection study in pure and LPS-sensitised hypoxic-ischemic piglets using the formulation of Example 2

Maternal inflammation/infection alone or in combination with birth asphyxia increases the risk for perinatal brain injury and hypothermia is not neuroprotective after infection-sensitized neonatal hypoxic-ischemic brain injury (Osredkar D et al., Resuscitation. 2014 Apr;85(4):567-72). We hypothesise that melatonin has better neuroprotection than hypothermia in LPS sensitised-HI due to its beneficial immunomodulatory effects. We propose to use E Coli lipopolysaccharide to induce a systemic inflammatory response; this endotoxin has previously been used in experimental models (Wang X et al., Ann Neurol. 2007; 61(3):263-71).

Hypoxia-ischemia will induced by remotely inflating the vascular occluders around both common-carotid arteries, and simultaneously reducing FiO2 to 6% v/v (Robertson N et al., Brain. 2013 Jan;136(Pt 1):90-105).

During hypoxia-ischemia, the β-NTP peak height will be continuously monitored through magnetic resonance for ensuring consistency in delivering the insult. Group of treatments are depicted in Figure 1. Pharmacokinetic modelling has shown that a dose of 15-18 mg/kg over 2-3h started at 1h after resuscitation, repeated every 24h, would lead to a Cmax at 3h, 4-5h earlier than the study reported in example 3. This would allow a time window of 1 hour for starting the intervention, which is compatible with the clinical practice.

Neuroprotective efficacy of melatonin is evaluated by: MRI/MRS will be acquired at 2, 24 and 48h after HI (1H MRS lactate/NAA our main biomarker). Continuous EEG and amplitude integrated EEG (aEEG), which is strongly predictive of outcome in infants with NE, is acquired between the MRI snapshot studies at 24 and 48h.

At 48h the experiment is terminated. Brain is perfusion-fixed for immunohistochemistry (TUNEL and/or cleaved caspase 3 and/or IBA-1) and discrete brain areas quantified to assess neuroprotection as well as modulatory effects against neuro inflammation.

Plasma melatonin PK levels 1, 2, 4, 6, 12, 18, 24, 36, 48h after HI are measured.

Assessment of immune modulatory effects of melatonin: by measuring plasma concentrations of pro-inflammatory mediators (for instance, IL-1α, IL-1β, IL-5, IL-6; TNF-α, IFNγ). Effect of LPS E Coli infusion on physiological parameters, MRS, MRI, EEG, NIRS, genetic and blood biomarkers are established.

The scheme of the protocol study is reported in Figure 7.

### EXAMPLE 6 - In vitro determination of the neuroprotective concentrations of melatonin

Concentration-response studies were aimed at detecting the range of concentration of melatonin resulting in neuroprotection against oxygen-glucose deprivation (OGD), an experimental model simulating hypoxia-ischemia and reperfusion. Ischemia was simulated in vitro by anoxia combined with a glucose-free media (OGD) in rat organotypic hippocampal slice cultures. Cell death, estimated by propidium iodide incorporation, induced by OGD was 90.8 ± 7.6% and was reduced to 64.3 ± 85.1%, 57.6 ± 6.9%, 60.9 ± 7.4%, 47.3 ± 3.8% and 38.9 ± 4.2%, after treatment with 1, 3, 10, 25 and 50 µM melatonin, respectively. Statistical analysis showed a significant effect of melatonin at all the doses tested (p<0.01 Dunnet's test after ANOVA), with the exception of 1 uM.

These results demonstrate that melatonin reduces OGD-induced cell death in hippocampal slice cultures of neonatal rats in a concentration-dependent manner. Pharmacokinetics studies performed in neonatal piglets (see Example 4) showed that infusion of 15 mg/Kg of melatonin formulation of Example 2 resulted in a range of plasma concentration of melatonin comprised between 16 and 4 mg/l (69-17 µM) which would be neuroprotective in OGD. Thus, intravenous infusion of melatonin at 15 mg/kg, allows to reach sustained and stable neuroprotective plasma concentration of melatonin which could counteract brain injury induced by neonatal hypoxia-ischemia.

## Claims

1. A safe pharmaceutical formulation comprising melatonin dissolved in an aqueous vehicle for use in the treatment of brain injuries caused by birth asphyxia in a neonate, wherein said formulation is administered by parenteral infusion at a daily dose of from 10 to 20 mg/kg, **characterized in that** the administration is started not earlier than 45 minutes but not later than 4 hours from resuscitation of said neonate and the time period for the infusion is comprised between 30 minutes and 4 hours.

2. The pharmaceutical formulation for use according to claim 1, wherein the disorder is selected from the group consisting of Perinatal Arterial Stroke (PAS), and Periventricular Leucomalakia (PVL), and hypoxic - ischemic encephalopathy (HIE).

3. The pharmaceutical formulation for use according to claim 2, wherein the disorder is hypoxic - ischemic encephalopathy (HIE).

4. The pharmaceutical formulation for use according to claim 3, wherein the administration is started not earlier than 1 hour but not later than 2 hours.

5. The pharmaceutical formulation for use according to claim 1, wherein the time period for the infusion is comprised between 1 hour and 2 hours.

6. The pharmaceutical formulation for use according to any one of the preceding claims, which is in form of a dry powder to be dissolved extemporaneously before use or in form of ready-to-use formulation.

7. The pharmaceutical formulation for use according to any one of the preceding claims, wherein the concentration of melatonin is comprised between 1 and 10 mg/ml.

8. The pharmaceutical formulation for use according to claim 7, wherein the concentration of melatonin is comprised between 2.5 and 5.0 mg/ml.

9. The pharmaceutical formulation for use according to any one of the preceding claims, wherein the pH is comprised between 6.5 and 7.5.

10. The pharmaceutical formulation for use according to any one of the preceding claims, wherein the osmolality is comprised between 250 and 350 mOsm/kg.

11. The pharmaceutical formulation for use according to any one of the preceding claims comprising sulfobutylether-β-cyclodextrin as solubilizing agent.

12. The pharmaceutical formulation for use according to any one of the preceding claims, comprising 2.5 or 5.0 mg/ml melatonin, 25 or 50 mg/ml sulfobutylether-β-cyclodextrin, 0.2 mg/ml phosphate buffer, optionally 4.5 mg/ml sodium chloride, and water for injection.

## Patentansprüche

1. Sichere pharmazeutische Formulierung, umfassend Melatonin, gelöst in einem wässrigen Vehikel, zur Verwendung bei der Behandlung von Hirnverletzungen, die durch Geburtsasphyxie bei einem Neugeborenen verursacht werden, wobei die Formulierung durch parenterale Infusion in einer täglichen Dosis von 10 bis 20 mg/kg verabreicht wird, **dadurch gekennzeichnet, dass** die Verabreichung nicht früher als 45 Minuten, aber nicht später als 4 Stunden nach der Wiederbelebung des Neugeborenen begonnen wird, und der Zeitraum für die Infusion zwischen 30 Minuten und 4 Stunden liegt.

2. Pharmazeutische Formulierung zur Verwendung nach Anspruch 1, wobei die Störung ausgewählt ist aus der Gruppe, bestehend aus Perinatalem Arteriellen Schlaganfall (PAS) und Periventrikulärer Leukomalakie (PVL) und hypoxisch-ischämischer Enzephalopathie (HIE).

3. Pharmazeutische Formulierung zur Verwendung nach Anspruch 2, wobei die Störung hypoxischischämische Enzephalopathie (HIE) ist.

4. Pharmazeutische Formulierung zur Verwendung nach Anspruch 3, wobei die Verabreichung nicht früher als 1 Stunde, aber nicht später als 2 Stunden begonnen wird.

5. Pharmazeutische Formulierung zur Verwendung nach Anspruch 1, wobei der Zeitraum für die Infusion zwischen 1 Stunde und 2 Stunden liegt.

6. Pharmazeutische Formulierung zur Verwendung nach einem der vorhergehenden Ansprüche, die in Form eines trockenen Pulvers, das vor der Verwendung extemporär aufzulösen ist, oder in Form einer gebrauchsfertigen Formulierung vorliegt.

7. Pharmazeutische Formulierung zur Verwendung nach einem der vorhergehenden Ansprüche, wobei die Konzentration von Melatonin zwischen 1 und 10 mg/ml liegt.

8. Pharmazeutische Formulierung zur Verwendung nach Anspruch 7, wobei die Konzentration von Melatonin zwischen 2,5 und 5,0 mg/ml liegt.

9. Pharmazeutische Formulierung zur Verwendung nach einem der vorhergehenden Ansprüche, wobei der pH-Wert zwischen 6,5 und 7,5 liegt.

10. Pharmazeutische Formulierung zur Verwendung nach einem der vorhergehenden Ansprüche, wobei die Osmolalität zwischen 250 und 350 mOsm/kg liegt.

11. Pharmazeutische Formulierung zur Verwendung nach einem der vorhergehenden Ansprüche, umfassend Sulfobutylether-β-cyclodextrin als Solubilisierungsmittel.

12. Pharmazeutische Formulierung zur Verwendung nach einem der vorhergehenden Ansprüche, umfassend 2,5 oder 5,0 mg/ml Melatonin, 25 oder 50 mg/ml Sulfobutylether-β-cyclodextrin, 0,2 mg/ml Phosphatpuffer, gegebenenfalls 4,5 mg/ml Natriumchlorid und Wasser zur Injektion.

## Revendications

1. Formulation pharmaceutique sans risque comprenant de la mélatonine dissoute dans un véhicule aqueux pour son utilisation dans le traitement de lésions cérébrales dues à une asphyxie néonatale chez un nouveau-né, dans laquelle ladite formulation est administrée par perfusion par voie parentérale à une dose quotidienne de 10 à 20 mg/kg, **caractérisée en ce que** l'administration est entamée pas avant 45 minutes, mais pas plus tard que 4 heures à compter de la réanimation dudit nouveau-né et le laps de temps pour la perfusion est compris entre 30 minutes et 4 heures.

2. Formulation pharmaceutique pour son utilisation selon la revendication 1, dans laquelle le trouble est choisi parmi le groupe constitué par un accident vasculaire cérébral artériel périnatal (PAS) et une leucomalacie périventriculaire (PVL), et par une encéphalopathie hypoxique-ischémique (HIE).

3. Formulation pharmaceutique pour son utilisation selon la revendication 2, dans laquelle le trouble est une encéphalopathie hypoxique-ischémique (HIE).

4. Formulation pharmaceutique pour son utilisation selon la revendication 3, dans laquelle l'administration est entamée pas avant 1 heure, mais pas plus tard que 2 heures.

5. Formulation pharmaceutique pour son utilisation selon la revendication 1, dans laquelle le laps de temps pour la perfusion est compris entre 1 heure et 2 heures.

6. Formulation pharmaceutique pour son utilisation selon l'une quelconque des revendications précédentes, qui se présente sous la forme d'une poudre sèche qui doit être dissoute de manière extemporanée avant son utilisation ou sous la forme d'une formulation prête à l'emploi.

7. Formulation pharmaceutique pour son utilisation selon l'une quelconque des revendications précédentes, dans laquelle la concentration de la mélatonine est comprise entre 1 et 10 mg/ml.

8. Formulation pharmaceutique pour son utilisation selon la revendication 7, dans laquelle la concentration de la mélatonine est comprise entre 2,5 et 5,0 mg/ml.

9. Formulation pharmaceutique pour son utilisation selon l'une quelconque des revendications précédentes, dans laquelle le pH est compris entre 6,5 et 7,5.

10. Formulation pharmaceutique pour son utilisation selon l'une quelconque des revendications précédentes, dans laquelle l'osmolalité est comprise entre 250 et 350 mOsm/kg.

11. Formulation pharmaceutique pour son utilisation selon l'une quelconque des revendications précédentes, qui comprend de la sulfobutyléther-β-cyclodextrine à titre d'agent de solubilisation.

12. Formulation pharmaceutique pour son utilisation selon l'une quelconque des revendications précédentes, qui comprend 2,5 ou 5,0 mg/ml de mélatonine, 25 ou 50 mg/ml de sulfobutyléther-β-cyclodextrine, 0,2 mg/ml d'un tampon de phosphate, de manière facultative 4,5 mg/ml de chlorure de sodium, et de l'eau pour préparations injectables.
